# EUROPEAN PATENT APPLICATION

(11) **EP 3 067 699 A1**
(43) Date of publication of application: **14.09.2016**
(21) Application number: 15382108.7
(22) Date of filing: 11.03.2015
(51) Int. Cl.: G01N 33/68

(54) **METHOD FOR DIAGNOSING ALZHEIMER'S DISEASE**

(71) Applicant: Neuron Bio, S.A., 18100 Armilla - Granada (ES)
(72) Inventor: Burgos Muñoz, Javier Santos, 18100 Armilla - Granada (ES); Santana Martínez, Soraya, 18100 Armilla - Granada (ES); Ramírez Moreno, Carlos, 18100 Armilla - Granada (ES)
(74) Representative: ABG Patentes, S.L.

(57) **Abstract**

The invention relates to methods for diagnosing Alzheimer's disease (AD) in a subject based on the determination of the levels of different biomarkers. The invention also provides for determining the likelihood that a subject develops AD based on the determination of the levels of the above biomarkers.

## Description

### FIELD OF THE INVENTION

The present invention falls within the field of diagnosis and, more specifically, it relates to the diagnosis of Alzheimer's disease based on the determination of the levels of different markers in biofluids.

### BACKGROUND OF THE INVENTION

Alzheimer's disease (AD) is a progressive degenerative disease of the central nervous system characterized by progressive and increasing memory loss, followed by loss of control of limbs and bodily functions and eventual death. It is by far the most common cause of dementia affecting 1 to 6% of people over the age of 65 years and between 10 to 20% of those over 80.

AD is distinguished from other types of dementia by several pathological features, including the progressive appearance in the brain of the patients of senile plaques in the extracellular space between neurons. The plaques have central cores of amyloid deposits formed mainly by fibrils of a 40-42 amino acids peptide referred to amyloid β peptide (Aβ) surrounded by degenerated neurons or parts thereof and glial cells. This peptide results from the proteolytic processing of a precursor protein called amyloid precursor protein (APP). AD can be classified according to the age of appearance as early onset (age under 60 years) and late onset (age above 60 years), according to the existence of an autosomic dominant inheritance, as familiar AD or sporadic AD. Early onset familiar forms of AD can be associated to known mutation in the genes coding for APP, presenilin 1 and presenilin 2 (located, respectively, on chromosomes 21, 14 and 1). These classifications are not mutually exclusive. The most frequent forms are sporadic late-onset forms.

In clinical praxis, diagnosis of AD is carried out using clinical criteria based on the presence of typical clinical hallmarks and the exclusion of other types of dementia using neuroimaging techniques and biofluid analysis. Using these criteria, diagnostic reliability is acceptable although, according to studies done using brain autopsy, between 10-20% of the patients diagnosed with AD suffered from a different disease. Moreover, the current diagnostic methods can only be carried out when the neurodegenerative process is so advanced that the patient suffers from severe dementia and the brain damages are so extensive that the number of therapeutic measures is limited. Definitive diagnosis requires pathologic examination of post-mortem brain tissue.

Therefore, there is a need for identifying new biomarkers for the diagnosis of AD which are sensitive and specific and which allow distinguishing cognitive impairment due to age from those associated with the early symptoms of the process, as well as to distinguish changes due to AD and due to other degenerative conditions.

Methods are known in the prior art to diagnose AD by detecting the levels of biomarkers present in the brain or cerebrospinal fluid (CSF) of patients. Different biomarkers have been characterised whose determination is carried out in CSF. CSF reflects directly the composition of the extracellular space of the central nervous system and thus, provides higher concentrations as biomarkers. However, CSF can only be retrieved by means of lumbar punction, which is not a routine diagnostic method easily accepted by patients suffering from dementia, let alone in patients with memory disorders. Thus, there is a need for AD biomarkers which can be detected in samples which can be non-invasively retrieved from the body.

Suitable AD biomarkers described in the prior art and which can be detected in plasma include (i) markers derived from the amyloid plaque, (ii) autoantibodies against Aβ o βAPP, (iii) inflammatory markers such IL-6, its receptor or gp130, C-reactive protein or oxidative stress (isoprostanes), (iv) markers of lipidic metabolism (apoE, oxysterols) and (v) vascular disease markers (homocysteine, lipoprotein b C1q).

WO 2011/143574 describes diagnostic methods for AD based on the determination of the levels of several markers in CSF (Table 2) or plasma (Table 9) in combination with other clinical features such as age and sex. The markers that can be used according to the disclosed diagnostic method are α-1 microglobulin, angiopoietin-2, apolipoprotein E, β2m, CXCL13, cortisol, E-selectin, FAS, fatty acid binding protein, HGF, IGF BP-2, interleukin 10, NT-Pro-BNP, osteopontin, pancreatic polypeptide, PAPP-A, resistin, stem cell factor, tenascin C, trombomodulin, TIMP-1, VCAM-1, VEGF and von Willebrand factor (Tables 7 y 8, claim 5).

Galimbeti et al. (2007, Eur J Neurology 14:e3-e4) describes that IP-10 has diagnostic value for AD when its levels are determined in CSF (p. e3, right col., second par.), but not when they are determined in serum (Table 1). This document appears to indicate that the lack of diagnostic value of IP-10 levels in serum is due to the low sensitivity of the detection method.

WO 2015/006489 describes a method of screening for neurological diseases which comprises measuring two or more biomarkers selected from IL7, TNFα, IL5, IL6, CRP, IL10, TNC, ICAM1, FVII, I309, TNFR1, A2M, TARC, eotaxin3, VCAM1, TPO, FABP, IL18, B2M, SAA, PPY, DJ1 and/or α-synuclein in a blood sample (p. 2 I. 2-30). Among the neurological diseases, AD is studied.

WO 2013/153461 describes several groups of markers with diagnostic value for AD which have altered levels in saliva (par. [0025]), including (i) cTnl, myoglobin, MMP-9, MMP-8, MMP-2, sICAM-1, MPO, IL-4 and/or IL-5; (ii) BNP, IL-Iα, IL-13, IL-6, IL-8, IL-10, TNF-α, IFN-γ, cTnl, VEGF, insulin, GLP-1 (active), GLP-1 (total), TREM1, leukotriene E4, Akt1, Aβ-40, Aβ-42, and Fas ligand; (iii) PSA, G-CSF, MIP-Iα, IL-22, IL-21, IL-15, IL-7, GM-CSF, IL-2, IL-12, IL-17α, IL-Iβ, MCP, and IL-32; and (iv) RANTES, sVCAM-1, sICAM-1, apolipoproteins Al, D and E, IMA, MPO, s. α-amilase, aspartate aminotransferase, lactate dehydrogenase, tissue factor activity, MCP-1, sCD-40, IGF-I and IGF-II.

Edwards et al. (2014, Arch Clin Neuropsychol 29:506) disclose serum markers for early detection of AD and for discriminating AD and mild cognitive impairment, based on multivariate models with CRP, SAA, ICAM, VCAM, A2M, B2M, FVII, TNC, CA125, eotaxin3, IL5, IL6, IL7, IL10, IL18, TARC, TNFa, FABP, I309, PPY and THPO, combined with age, sex, education, APOE4 and animal fluency (Abstract).

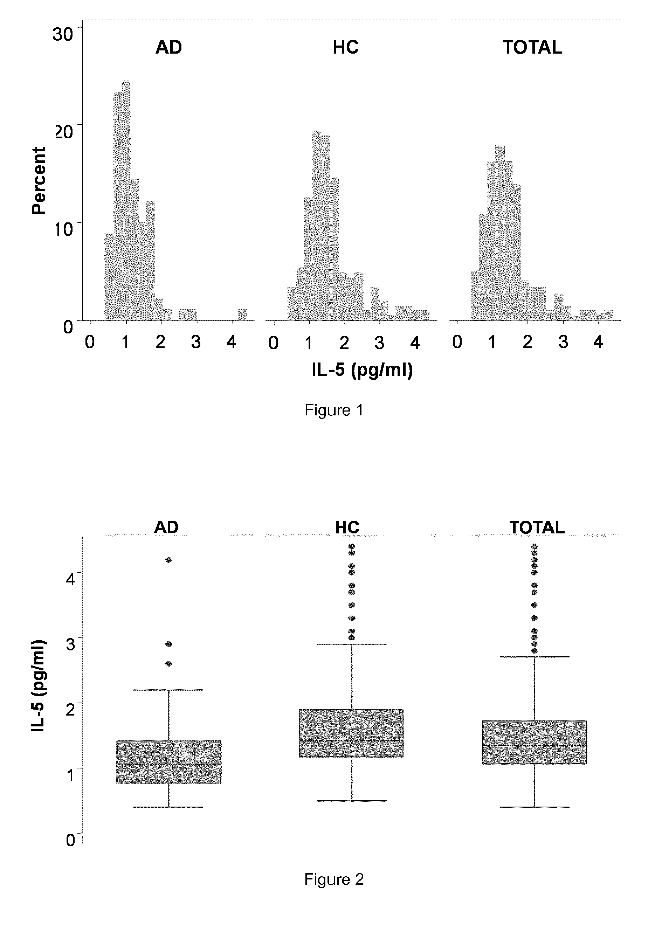

Richartz et al. (2005, J Psych Res 39:535-43) discloses the discrimination of AD's patients from healthy subjects based on the levels of IL-5 released in blood samples cultured with mitogens (PHA and LPS) (p. 539, par. bridging both columns; Fig. 2). However, this result is obtained by means of artificial stimulating the blood cells with mitogens, a state which does not resemble the real condition in AD's patients.

Despite the efforts made to date, there still exists a long-felt and continuing need in the art for reliable and cost-effective methods useful for diagnosing and predicting AD based on the analysis of bodily fluids, which can be easily obtainable by non-invasive means.

### SUMMARY OF THE INVENTION

The inventors of the present invention have observed that the determination of the levels of different biomarkers allow the identification of subjects with AD. The panel of biomarkers identified by the inventors is useful for diagnosing AD and for identifying a subject without dementia which is at high risk of developing AD. Surprisingly, the methods developed by the inventors also have diagnostic and predictive value in biofluid samples obtained non-invasively from the body.

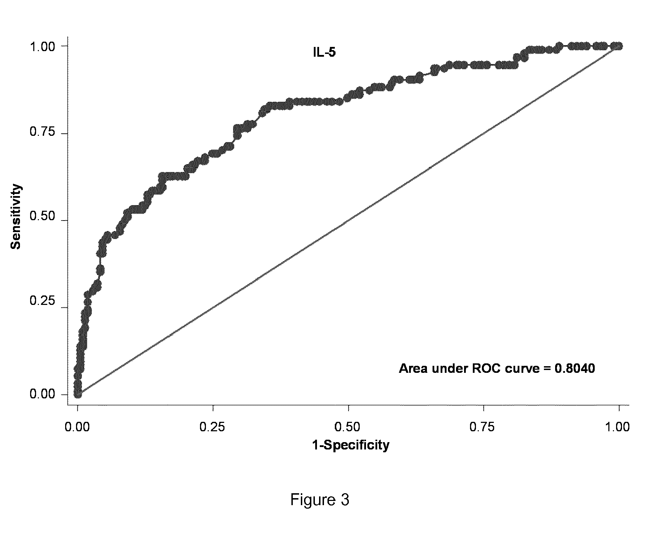

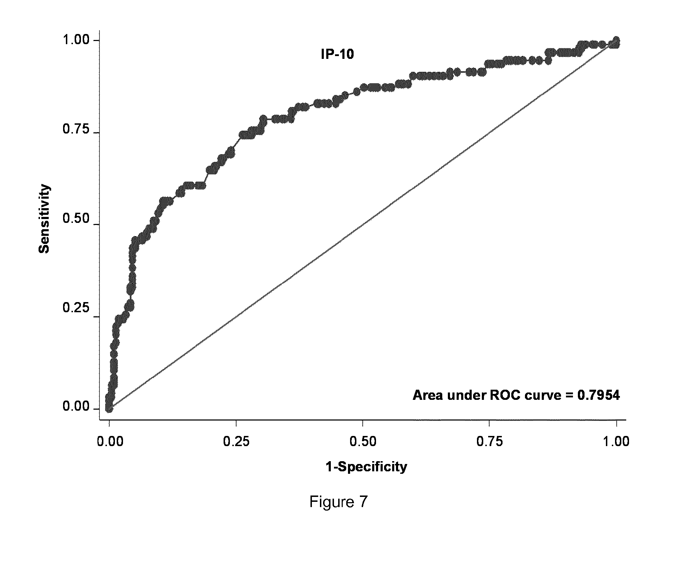

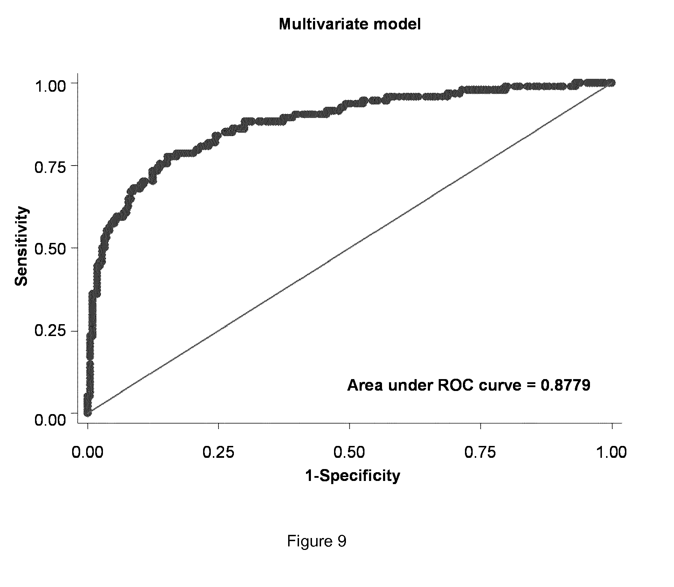

In particular, as illustrated by the examples of the application, the inventors have shown that the levels of the markers IP-10 and/or IL-5 are able to discriminate AD's patients from healthy controls with an Area Under Curve (AUC) of ROC curve to 0.7954 and 0.8040, respectively (figures 7 and 3, respectively). The discrimination power of the model can be significantly improved by combining these two markers and by increasing the number of markers of the signature and adjusting by demographic factors, reaching an AUC of 0.8779 when five biomarkers are determined (Figure 9).

Based on the previous findings, the following inventive aspects have been developed.

In a first aspect, the invention relates to an *in vitro* method for diagnosing Alzheimer's disease (AD) in a subject comprising:
i) determining in a biofluid sample from said subject the level of IP-10, and
ii) comparing the level of IP-10 with a reference value,
wherein said biofluid is not cerebrospinal fluid (CSF), and wherein decreased levels of IP-10 compared to the reference value is indicative of said subject suffering from AD.

In a second aspect, the invention relates to an *in vitro* method for diagnosing AD in a subject comprising:
i) determining in a biofluid sample from said subject the level of IL-5, and
ii) comparing the level of IL-5 with a reference value,
wherein decreased levels of IL-5 compared to the reference value is indicative of said subject suffering from AD.

In a third aspect, the invention relates to an *in vitro* method for diagnosing AD in a subject comprising:
i) determining in a first biofluid sample from said subject the level of IP-10,
ii) determining in a second biofluid sample from said subject the level of IL-5, and
iii) comparing the level of IP-10 and IL-5 with their corresponding reference values,
wherein said first biofluid is not CSF, wherein said first biofluid sample and said second biofluid sample are the same or different, and wherein decreased levels of IP-10 compared to its reference value and decreased levels of IL-5 compared to its reference value is indicative of said subject suffering from AD.

In a fourth aspect, the invention relates to an *in vitro* method for determining the likelihood that a subject develops AD, comprising:
i) determining in a biofluid sample from said subject the level of IP-10, and
ii) comparing the level of IP-10 with a reference value,
wherein said biofluid is not CSF, and wherein decreased levels of IP-10 compared to the reference value is indicative of a high likelihood of the subject developing AD.

In a fifth aspect, the invention relates to an *in vitro* method for determining the likelihood that a subject develops AD, comprising:
i) determining in a biofluid sample from said subject the level of IL-5, and
ii) comparing the level of IL-5 with a reference value,
wherein decreased levels of IL-5 compared to the reference value is indicative of a high likelihood of the subject developing AD.

In a sixth aspect, the invention relates to an *in vitro* method for determining the likelihood that a subject develops AD, comprising:
i) determining in a first biofluid sample from said subject the level of IP-10,
ii) determining in a second biofluid sample from said subject the level of IL-5, and
iii) comparing the level of IP-10 and IL-5 with their corresponding reference values,
wherein said first biofluid is not CSF, wherein said first biofluid sample and said second biofluid sample are the same or different, and wherein decreased levels of IP-10 compared to its reference value and decreased levels of IL-5 compared to its reference value is indicative of a high likelihood of the subject developing AD.

In a seventh aspect, the invention relates to a method for the treatment of AD in a subject in need thereof, which comprises the administration to the subject a drug useful for the treatment of AD, wherein the subject has been selected by a method according to the first, second or third aspects of the invention.

In an eighth aspect, the invention relates to a method for the prevention of AD in a subject in need thereof, which comprises the administration to the subject of a drug useful for the prevention of AD, wherein the subject has been selectedby a method according to the fourth, fifth or sixth aspects of the invention.

### BRIEF DESCRIPTION OF THE FIGURES

Figure 1. Histogram of frequencies with the distribution of IL-5 levels in the groups. The histograms have been truncated on the 5 pg/ml to allow for better viewing.
Figure 2. Graphics boxes with the distribution of IL-5 levels in the groups. Graphics have been truncated on the 5 pg/ml to allow for better viewing.
Figure 3. ROC curve of IL-5 marker.
Figure 4. Model calibration for IL-5 as diagnostic marker by Hosmer-Lemeshow test.
Figure 5. Histogram of frequencies with the distribution of IP-10 levels in the groups. The histograms have been truncated on the 2.000 pg/ml to allow for better viewing.
Figure 6. Graphics boxes with the distribution of IP-10 levels in the groups. Graphics have been truncated on the 2.000 pg/ml to allow for better viewing.
Figure 7. ROC curve of IP-10 marker.
Figure 8. Model calibration for IP-10 as diagnostic marker by Hosmer-Lemeshow test.
Figure 9. ROC curve of multivariate model.
Figure 10. Model calibration for multivariate model by Hosmer-Lemeshow test.

### DETAILED DESCRIPTION OF THE INVENTION

### 1. Methods for diagnosing Alzheimer's disease in a subject

The expression "method for diagnosing Alzheimer's disease", as used herein, refers to a method that consists essentially of the steps of the diagnostic methods of the invention although it may include additional steps. "Diagnosing", as used herein, refers to evaluating the probability according to which a subject suffers from a disease, in particular from Alzheimer's disease. The method for the diagnosis of Alzheimer's disease of the invention is carried out *in vitro.*

As will be understood by those skilled in the art, the diagnosis, although preferred to be, need not be correct for 100% of the subjects to be diagnosed or evaluated. The term, however, requires that a statistically significant portion of subjects can be identified as suffering from AD. Whether a subject is statistically significant can be determined without further ado by the person skilled in the art using various well known statistic evaluation tools, e.g., determination of confidence intervals, p-value determination, cross-validated classification rates and the like etc. Preferred confidence intervals are at least 50%, at least 60%, at least 70%, at least 80%, at least 90% or at least 95%. The p-values are, preferably, 0.05, 0.01, 0.005 or lower.

### 1.1. First diagnostic method

In a first aspect, the invention relates to an *in vitro* method for diagnosing Alzheimer's disease (AD) in a subject, hereinafter "the first diagnostic method of the invention", comprising:
i) determining in a biofluid sample from said subject the level of IP-10, and
ii) comparing the level of IP-10 with a reference value,
wherein said biofluid is not cerebrospinal fluid (CSF), and
wherein decreased levels of IP-10 compared to the reference value is indicative of said subject suffering from AD.

The term "diagnosing", as used herein, refers to evaluating the probability according to which a subject suffers from a disease, in particular from AD. The method for diagnosing AD is carried out *in vitro.*

The term "Alzheimer's disease" or "AD" or "Alzheimer's", as used herein, refers a mental deterioration associated with specific degenerative brain disease that is characterized by senile plaques, neuritic tangles and progressive neuronal loss which manifests clinically in progressive memory deficits, confusion, behavioural problems, inability to care for oneself, gradual physical deterioration and, ultimately, death. Typically, patients suffering from AD are identified using the NINCDS-ADRDA (National Institute of Neurological and Communicative Disorders and the Alzheimer's Disease and Related Disorders Association) criteria: Clinical Dementia Rating (CDR) = 1; Mini Mental State Examination (MMSE) = 16-24 points and Medial temporal atrophy (determined by Magnetic Resonance Imaging, MRI) >3 points in Scheltens scale.

As used herein, the term AD is intended to include all the stages of the disease, including, without limitation, the following stages defined by NINCDS-ADRDA Alzheimer's Criteria for diagnosis in 1984 (McKhann et al., 1984, Neurology 34:939-44):
- Definite AD: The patient meets the criteria for probable AD and has histopathological evidence of AD via autopsy or biopsy.
- Probable or prodromal AD: Dementia has been established by clinical and neuropsychological examination. Cognitive impairments also have to be progressive and be present in two or more areas of cognition. The onset of the deficits has been between the ages of 40 and 90 years and finally there must be an absence of other diseases capable of producing a dementia syndrome.
- Possible or non-prodromal AD: There is a dementia syndrome with an atypical onset, presentation or progression; and without a known etiology; but no co-morbid diseases capable of producing dementia are believed to be in the origin of it.

Additionally, the National Institute on Aging and the Alzheimer's Association revised in 2011 the diagnostic criteria into the NIA-AA's Criteria for Alzheimer's disease (Albert et al., 2011, Alzheimer's Dement 7:270-9; Jack et al., 2011, Alzheimer's Dement 7:257-62; McKhann et al., 2011, Alzheimer's Dement 7:263-9; Sperling et al., 2011, Alzheimer's Dement 7:280-92). The term AD is also intended to include the stages defined by these criteria.

The term "subject", as used herein, refers to all animals classified as mammals and includes, without limitation, domestic and farm animals, primates and humans, e.g., human beings, non-human primates, cows, horses, pigs, sheep, goats, dogs, cats, or rodents. Preferably, the subject is a male or female human of any age or race.

In a particular embodiment of the first diagnostic method of the invention, the subject is a nondemented subject with cognitive impairment.

In a preferred embodiment, the nondemented subject with cognitive impairment is a subject suffering from prodromal AD or mild cognitive impairment (MCI).

The term "prodromal AD" or "mild cognitive impairment with probable AD" or "preclinical AD", as used herein, refers to subjects showing MCI and which are considered as showing high risk for conversion to AD. Criteria for identifying a subject as prodromal AD are those as defined by the NIA-AA's criteria (Sperling et al., 2011, cited *supra).*

The term "mild cognitive impairment" or "MCI" is related to a transitional stage of cognitive impairment between normal aging and dementia. Subjects are usually identified as having MCI if they fulfil the Mayo Clinic criteria (Clinical Dementia Rating, CDR = 0.5), they show a medial temporal atrophy (determined by MRI) which is higher than 3 points in Scheltens scale, they show a pattern of parietal and/or temporal hypometabolism in PET with 18-fluorodeoxyglucose (PET-FDG) (suggestive of AD). Alternatively, MCI can also be identified following the NIA-AA's criteria (Albert et al., 2011, cited *supra).*

The first step of the first diagnostic method of the invention comprises the determination in a biofluid sample from said subject of the level of IP-10.

In a particular embodiment, the first step of the first diagnostic method of the invention further comprises the determination in said biofluid sample from said subject of the levels of at least one additional biomarker, said biomarker being IL-12.

In another particular embodiment, the first step of the first diagnostic method of the invention further comprises the determination in said biofluid sample from said subject of the levels of at least one additional biomarker, said biomarker being RANTES.

In another particular embodiment, the first step of the first diagnostic method of the invention further comprises the determination in said biofluid sample from said subject of the levels of at least one additional biomarker, said biomarker being EGF.

In a preferred embodiment, the first step of the first diagnostic method of the invention further comprises the determination in said biofluid sample from said subject of the levels of at least two additional biomarkers, said two additional biomarkers being IL-12 and RANTES.

In a preferred embodiment, the first step of the first diagnostic method of the invention further comprises the determination in said biofluid sample from said subject of the levels of at least two additional biomarkers, said two additional biomarkers being IL-12 and EGF.

In a preferred embodiment, the first step of the first diagnostic method of the invention further comprises the determination in said biofluid sample from said subject of the levels of at least two additional biomarkers, said two additional biomarkers being RANTES and EGF.

In a more preferred embodiment, the first step of the first diagnostic method of the invention further comprises the determination in said biofluid sample from said subject of the levels of IL-12, RANTES and EGF.

The term "biofluid sample", as used herein, refers to any sample collected any bodily fluid or liquid derived from the body of a living organism. Suitable samples for use in the first diagnostic method of the invention include any bodily fluid selected from the group consisting of blood, serum, plasma, CSF, saliva, gingival crevicular fluid, urine, seminal plasma, tears, and nipple fluid. In the context of the first diagnostic method of the invention, the biofluid is not CSF. Preferably, the bodily fluid sample is blood, plasma, blood serum or saliva.

The term "IP-10", as used herewith, refers to a small cytokine belonging to the CXC chemokine family that is secreted by several cell types, expression can be highly induced in a variety of cells, including endothelial cells, keratinocytes, fibroblasts, mesangial cells, astrocytes, monocytes, and neutrophils by stimulation with IFN-α, IFN-β, IFN-γ or LPS and in T cells by antigen activation. It is also known as interferon gamma-induced protein 10, C-X-C motif chemokine 10 and CXCL10. The human IP-10 is depicted under UniProt accession number P02778.2, as of 5^{th} March, 2015.

The term "IL-12", as used herewith, refers to a 70-kDa pleiotropic heterodimeric cytokine composed of two disulfide-bound subunits designated p35 (35 kDa) and p40 (40 kDa). IL-12A, also known as interleukin 12A, p35, CLMF1, NFSK, NKSF1, encodes the subunit p35. Human IL-12A is depicted under UniProt accession number P29459, as of 5^{th} March, 2015. IL-12B, also known as interleukin 12B, p40, CLMF2, NKSF, IMD28, IMD29 and NKSF2, encodes the subunit p40. Human IL-12A is depicted under UniProt accession number P29460, as of 5th March, 2015.

The term "RANTES", as used herewith, refers to chemokine (C-C motif) ligand 5, a chemotactic cytokine or chemokine that is chemotactic for T cells, eosinophils, and basophils, and plays an active role in recruiting leukocytes into inflammatory sites. It is also known as CCL5, D17S136E, SCYA5, SIS-delta, SISd, TCP228, and eoCP. Human RANTES is depicted under UniProt accession number P13501, as of 5^{th} March, 2015.

The term "EGF", as used herewith, refers to 53 amino acid growth factor that stimulates cell growth, proliferation, and differentiation by binding to its receptor EGFR. It is also known as HOMG4 and URG. The human EGF is depicted under GenBank accession number AFA26280.1, as of 5^{th} March, 2015, and its protein precursor is depicted under UniProt accession number P01133, as of 5^{th} March, 2015.

The term "level", as used herein, refers to the quantity of a biomarker detectable in a sample. The methods for determining the level of the biomarkers according to the first diagnostic method of the invention will depend on the type of biomarker and are well known in the art. The level of said biomarker can be determined by any method known in the art suitable for the determination and quantification of a protein in a sample.

In a particular embodiment, the determination of the level of a biomarker is carried out by means of an immunoassay. In a preferred embodiment, the immunoassay is a chemiluminiscent enzyme immunoassay, more preferably a solid-phase chemiluminiscent enzyme immunoassay. Suitable immunoassays include, without limitation, Western blot, immunoprecipitation, enzyme-linked immunosorbent assay (ELISA), inmunoturbidimetry, surface plasmon resonance (SPR), radioimmunoassay (RIA), chemiluminiscent enzyme immunoassay, and immunology multiplex assay.

The second step of the first diagnostic method of the invention comprises comparing the level of IP-10 with its reference value.

In particular embodiments, as aforementioned, the first diagnostic method of the invention further comprises comparing the level of said at least one additional biomarker selected from IL-12, RANTES and EGF with its reference value.

The term "reference value", as used in the context of the diagnostic methods of the invention, relates to a predetermined criteria used as a reference for evaluating the values or data obtained from the samples collected from a subject. The reference value or reference level can be an absolute value, a relative value, a value that has an upper or a lower limit, a range of values, an average value, a median value, a mean value, or a value as compared to a particular control or baseline value. The reference value according to the diagnostic methods of the invention can obtained from one or more subjects who do not suffer from AD (i.e., control or healthy subjects). A subject is considered as not suffering from AD according to NINCDS-ADRDA criteria (McKhann G. et al., 1984, cited *supra*); particularly using the tests outlined in Table 2, or any other current or future medical criteria.

According to the first diagnostic method of the invention, decreased levels of IP-10 compared to the reference value is indicative of said subject suffering from AD.

In particular embodiments, as aforementioned, in addition to decreased levels of IP-10 compared to the reference value, decreased levels of IL-12 and/or RANTES and/or EGF compared to their respective reference value is indicative of said subject suffering from AD.

According to the invention, the level of a biomarker is considered "decreased" when the level of said biomarker in a sample is lower than a reference value. The levels of a biomarker are considered to be lower than its reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than its reference value.

Likewise, in the context of the invention, the level of a biomarker is considered "increased" when the level of said biomarker in a sample is higher than a reference value. The levels of a biomarker are considered to be higher than its reference value when it is at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than its reference value.

In another particular embodiment, the first diagnostic method of the invention further comprises determining at least one demographic factor selected from age, sex and education level.

According to the first diagnostic method of the invention, in addition to decreased levels of IP-10 compared to the reference value, increased age and/or female gender and/or a low level of education is also indicative of said subject suffering from AD.

In a preferred embodiment, increased age (as years) presents an odds ratio (OD) of 1.10 for the diagnosis of AD.

In another preferred embodiment, female presents an OD of 2.25 in respect to male for the diagnosis of AD.

In another preferred embodiment, a level of education lower than basic education is also indicative of said subject suffering from AD.

In another particular embodiment, the first diagnostic method of the invention further comprises determining the presence or absence of a nucleic acid sequence encoding apolipoprotein E type 4 isoform (ApoE4) in a sample containing nucleic acid from said subject. Accordingly, the presence of a nucleic acid sequence encoding ApoE4 is indicative of the subject suffering from AD.

The term "apolipoprotein E" or "ApoE", as used herein, refers to a protein found in chylomicrons and intermediate-density lipoproteins (IDLs) that is essential for the normal catabolism of triglyceride-rich lipoprotein constituents. In humans, Apolipoprotein E is encoded by the gene *ApoE,* which is a polymorphic gene with three major alleles, *ε*2, *ε*3, and *ε*4, which encodes the isoforms ApoE2 (cys112, cys158), ApoE3 (cys112, arg158), and ApoE4 (arg112, arg158). Human Apo E is depicted with the UniProt accession number P02649, as of 16^{th} April, 2014.

The term "apolipoprotein E type 4 isoform" or "apolipoprotein E epsilon 4" or "ApoE4", as used herein, refers to the isoform E4 of the protein apolipoprotein E, which is defined by the presence of the residue arginine in positions 112 and 158 of the amino acid sequence.

The presence or absence of a nucleic acid sequence encoding ApoE4 can be determined in any sample containing nucleic acid taken form the subject. It will be understood that the sample may be the same biofluid sample as the first step of the first diagnostic method, or a different sample, which may be a tissue or biofluid sample, so long as the sample contains nucleic acid from said subject.

The presence or absence of a nucleic acid encoding ApoE4 can be determined in a sample from the patient by any method known in the art suitable for the determination of the presence of a nucleic acid encoding ApoE4. Determining the presence or absence of nucleic acid encoding an ApoE4 isoform may be carried out with an oligonucleotide probe labelled with a suitable detectable group, or by means of an amplification reaction such as a polymerase chain reaction or ligase chain reaction (the product of which amplification reaction may then be detected with a labelled oligonucleotide probe or a number of other techniques). DNA amplification techniques such as the foregoing can involve the use of a probe, a pair of probes, or two pairs of probes which specifically bind to DNA encoding ApoE4, but do not bind to DNA encoding ApoE2 or ApoE3 under the same hybridization conditions, and which serve as the primer or primers for the amplification of the ApoE4 DNA or a portion thereof in the amplification reaction. In general, an oligonucleotide probe which is used to detect DNA encoding ApoE4 is an oligonucleotide probe which binds to DNA encoding ApoE4, but does not bind to DNA encoding ApoE2 or ApoE3 under the same hybridization conditions.

According to this particular embodiment, the presence of a nucleic acid sequence encoding ApoE4 in said sample is indicative of said subject suffering from AD.

### 1.2. Second diagnostic method

In a second aspect, the invention relates to an *in vitro* method for diagnosing Alzheimer's disease (AD) in a subject, hereinafter "the second diagnostic method of the invention", comprising:
i) determining in a biofluid sample from said subject the level of IL-5, and
ii) comparing the level of IL-5 with a reference value,
wherein decreased levels of IL-5 compared to the reference value is indicative of said subject suffering from AD.

The terms "diagnosing", "Alzheimer's disease", "subject", "biofluid sample", "level", and "reference value" have been described in detail in the context of the first diagnostic method of the invention and their definitions and particularities apply equally to the second diagnostic method of the invention.

In a particular embodiment of the second diagnostic method of the invention, the subject is a subject suffering from prodromal AD or MCI.

The first step of the second diagnostic method of the invention comprises the determination in a biofluid sample from said subject of the level of IL-5.

The term "IL-5", as used herewith, refers to the cytokine interleukin-5, which is produced by T helper-2 cells and mast cells and acts as a growth and differentiation factor for both B cells and eosinophils. It is also known as B-cell differentiation factor I, eosinophil differentiation factor, EDF, T-cell replacing factor, and TRF. The human IL-5 is depicted under UniProt accession number P05113, as of 5^{th} March, 2015.

In a particular embodiment, the first step of the second diagnostic method of the invention further comprises the determination in said biofluid sample from said subject of the levels of at least one additional biomarker, said biomarker being IL-12.

In another particular embodiment, the first step of the second diagnostic method of the invention further comprises the determination in said biofluid sample from said subject of the levels of at least one additional biomarker, said biomarker being RANTES.

In another particular embodiment, the first step of the second diagnostic method of the invention further comprises the determination in said biofluid sample from said subject of the levels of at least one additional biomarker, said biomarker being EGF.

In a preferred embodiment, the first step of the second diagnostic method of the invention further comprises the determination in said biofluid sample from said subject of the levels of at least two additional biomarkers, said two additional biomarkers being IL-12 and RANTES.

In a preferred embodiment, the first step of the second diagnostic method of the invention further comprises the determination in said biofluid sample from said subject of the levels of at least two additional biomarkers, said two additional biomarkers being IL-12 and EGF.

In a preferred embodiment, the first step of the second diagnostic method of the invention further comprises the determination in said biofluid sample from said subject of the levels of at least two additional biomarkers, said two additional biomarkers being RANTES and EGF.

In a more preferred embodiment, the first step of the second diagnostic method of the invention further comprises the determination in said biofluid sample from said subject of the levels of IL-12, RANTES and EGF.

The terms "IL-12", "RANTES" and "EGF" have been described in detail in the context of the first diagnostic method of the invention and their definitions and particularities apply equally to the second diagnostic method of the invention.

The level of biomarkers can be determined by any method known in the art suitable for the determination and quantification of a protein in a sample.

In a particular embodiment, the determination of the level of a biomarker is carried out by means of an immunoassay. In a preferred embodiment, the immunoassay is a chemiluminiscent enzyme immunoassay, more preferably a solid-phase chemiluminiscent enzyme immunoassay. These techniques have been described in detail in the context of the first diagnostic method of the invention.

The second step of the second diagnostic method of the invention comprises comparing the level of IL-5 with its reference value.

In particular embodiments, as aforementioned, the second diagnostic method of the invention further comprises comparing the level of said at least one additional biomarker selected from IL-12, RANTES and EGF with its reference value.

According to the second diagnostic method of the invention, decreased levels of IL-5 compared to the reference value is indicative of said subject suffering from AD.

In particular embodiments, as aforementioned, in addition to decreased levels of IL-5 compared to the reference value, decreased levels of IL-12 and/or RANTES and/or EGF compared to their respective reference value is indicative of said subject suffering from AD.

"Decreased" and "increased" levels of a biomarker have been described in detail in the context of the first diagnostic method of the invention.

In another particular embodiment, the second diagnostic method of the invention further comprises determining at least one demographic factor selected from age, sex and education level.

According to the second diagnostic method of the invention, in addition to decreased levels of IL-5 compared to the reference value, increased age and/or female gender and/or a low level of education is also indicative of said subject suffering from AD.

In a preferred embodiment, increased age (as years) presents an odds ratio (OD) of 1.08 for the diagnosis of AD.

In another preferred embodiment, female presents an OD of 2.06 in respect to male for the diagnosis of AD.

In another preferred embodiment, a level of education lower than basic education is also indicative of said subject suffering from AD.

In another particular embodiment, the second diagnostic method of the invention further comprises determining the presence or absence of a nucleic acid sequence encoding ApoE4 in a sample containing nucleic acid from said subject. Accordingly, the presence of a nucleic acid sequence encoding ApoE4 is indicative of the subject suffering from AD.

The term "ApoE4" has been described in detail in the context of the first diagnostic method of the invention and its particularities are equally applicable to the second diagnostic method of the invention.

### 1.3. Third diagnostic method

In a third aspect, the invention relates to an *in vitro* method for diagnosing Alzheimer's disease (AD) in a subject, hereinafter "the third diagnostic method of the invention", comprising:
i) determining in a first biofluid sample from said subject the level of IP-10,
ii) determining in a second biofluid sample from said subject the level of IL-5, and
iii) comparing the level of IP-10 and IL-5 with their corresponding reference values,
wherein steps (i) and (ii) can be performed in any order,
wherein said first biofluid is not CSF,
wherein said first biofluid sample and said second biofluid sample are the same or different, and
wherein decreased levels of IP-10 compared to its reference value and decreased levels of IL-5 compared to its reference value is indicative of said subject suffering from AD.

The terms "diagnosing", "Alzheimer's disease", "subject", "biofluid sample", "level", "reference value", "IP-10" and "IL-5" have been described in detail in the context of the first diagnostic method of the invention and their definitions and particularities apply equally to the second diagnostic method of the invention.

In a particular embodiment of the third diagnostic method of the invention, the subject is a subject suffering from prodromal AD or MCI.

The first step (i) of the third diagnostic method of the invention comprises the determination in a biofluid sample from said subject of the level of IP-10.

The second step (ii) of the third diagnostic method of the invention comprises the determination in a biofluid sample from said subject of the level of IL-5.

The first and second steps of the third diagnostic method of the invention can be performed in any order. Accordingly, the first step (i) can be carried out before the second step (ii), or the second step (ii) can be carried out before the first step (i).

The term "first biofluid sample", as used in the context of the third diagnostic method of the invention, refers to any biofluid sample with the exemption of CSF. Preferably, the first biofluid sample is blood, plasma, blood serum or saliva.

It will be recognisable for the person skilled in the art that said first biofluid sample may be the same as said second biofluid sample or, alternatively, that said first biofluid sample may be different from said second biofluid sample.

In a particular embodiment, the first step of the third diagnostic method of the invention further comprises the determination in said biofluid sample from said subject of the levels of at least one additional biomarker, said biomarker being IL-12.

In another particular embodiment, the first step of the third diagnostic method of the invention further comprises the determination in said biofluid sample from said subject of the levels of at least one additional biomarker, said biomarker being RANTES.

In another particular embodiment, the first step of the third diagnostic method of the invention further comprises the determination in said biofluid sample from said subject of the levels of at least one additional biomarker, said biomarker being EGF.

In a preferred embodiment, the first step of the third diagnostic method of the invention further comprises the determination in said biofluid sample from said subject of the levels of at least two additional biomarkers, said two additional biomarkers being IL-12 and RANTES.

In a preferred embodiment, the first step of the third diagnostic method of the invention further comprises the determination in said biofluid sample from said subject of the levels of at least two additional biomarkers, said two additional biomarkers being IL-12 and EGF.

In a preferred embodiment, the first step of the third diagnostic method of the invention further comprises the determination in said biofluid sample from said subject of the levels of at least two additional biomarkers, said two additional biomarkers being RANTES and EGF.

In a more preferred embodiment, the first step of the third diagnostic method of the invention further comprises the determination in said biofluid sample from said subject of the levels of IL-12, RANTES and EGF.

The terms "IL-12", "RANTES" and "EGF" have been described in detail in the context of the first diagnostic method of the invention and their definitions and particularities apply equally to the second diagnostic method of the invention.

The level of biomarkers can be determined by any method known in the art suitable for the determination and quantification of a protein in a sample.

In a particular embodiment, the determination of the level of a biomarker is carried out by means of an immunoassay. In a preferred embodiment, the immunoassay is a chemiluminiscent enzyme immunoassay, more preferably a solid-phase chemiluminiscent enzyme immunoassay. These techniques have been described in detail in the context of the first diagnostic method of the invention.

The third step (iii) of the third diagnostic method of the invention comprises comparing the level of IP-10 and IL-5 with their corresponding reference values.

In particular embodiments, as aforementioned, the third diagnostic method of the invention further comprises comparing the level of said at least one additional biomarker selected from IL-12, RANTES and EGF with its reference value.

According to the third diagnostic method of the invention, decreased levels of IP-10 compared to its reference value and decreased levels of IL-5 compared to its reference value is indicative of said subject suffering from AD.

In particular embodiments, as aforementioned, in addition to decreased levels of IP-10 compared to the reference value and decreased levels of IL-5 compared to the reference value, decreased levels of IL-12 and/or RANTES and/or EGF compared to their respective reference value is indicative of said subject suffering from AD.

"Decreased" and "increased" levels of a biomarker have been described in detail in the context of the first diagnostic method of the invention.

In another particular embodiment, the third diagnostic method of the invention further comprises determining at least one demographic factor selected from age, sex and education level.

According to the third diagnostic method of the invention, in addition to decreased levels of IP-10 compared to the reference value and decreased levels of IL-5 compared to the reference value, increased age and/or female gender and/or a low level of education is also indicative of said subject suffering from AD.

In a preferred embodiment, increased age (as years) presents an odds ratio (OD) of 1.10 for the diagnosis of AD.

In another preferred embodiment, female presents an OD of 2.04 in respect to male for the diagnosis of AD.

In another preferred embodiment, a level of education lower than basic education is also indicative of said subject suffering from AD.

In another particular embodiment, the third diagnostic method of the invention further comprises determining the presence or absence of a nucleic acid sequence encoding said ApoE4 in a sample containing nucleic acid from said subject. Accordingly, the presence of a nucleic acid sequence encoding ApoE4 is indicative of the subject suffering from AD.

The term "ApoE4" has been described in detail in the context of the first diagnostic method of the invention and its particularities are equally applicable to the third diagnostic method of the invention.

### 2. Methods for determining the likelihood that a subject develops AD

The term "method for determining the likelihood", as used herein, refers to a method for determining the probability of a particular event. In the context of the predictive methods of the invention, determining the likelihood that a subject develops AD refers to determining whether said patient has a high likelihood of developing AD. The term "high likelihood", as used herein, refers to a significant probability of developing AD. In a particular embodiment, a high likelihood is at least about 20%, including but not limited to about 25%, 30%, 35%, 40%, 45%, 50%, 55%, 60%, 65%, 70%, 75%, 80%, 85%, 90%, 95%, 100%, 150%, 200%, 300%, 400%, 500%, 600%, 700%, 800%, 900%, 1000%, and 1500%. In one particular embodiment, a high likelihood is at least 100%. In other embodiments, a high likelihood is at least 200%, at least 300%, at least 400%, at least 500%, at least 700%, at least 800%, at least 900% and at least 1000%. Other cut-offs or ranges as deemed suitable by the person skilled in the art to characterise the invention are however also contemplated, and those are also within scope of the present invention.

### 2.1. First predictive method

In another aspect, the invention relates to an *in vitro* method for determining the likelihood that a subject develops AD, hereinafter "the first predictive method of the invention", comprising:
i) determining in a biofluid sample from said subject the level of IP-10, and
ii) comparing the level of IP-10 with a reference value,
wherein said biofluid is not CSF, and
wherein decreased levels of IP-10 compared to the reference value is indicative of a high likelihood of the subject developing AD.

The terms "Alzheimer's disease", "subject", "biofluid sample", "level", and "IP-10" have been described in detail in the context of the diagnostic methods of the invention and their definitions and particularities apply equally to the first predictive method of the invention.

In a particular embodiment of the first predictive method of the invention, the subject is a nondemented subject with cognitive impairment.

In a preferred embodiment, the nondemented subject with cognitive impairment is a subject suffering from prodromal AD or MCI.

The first step of the first predictive method of the invention comprises the determination in a biofluid sample from said subject of the level of IP-10.

In a particular embodiment, the first step of the first predictive method of the invention further comprises the determination in said biofluid sample from said subject of the levels of at least one additional biomarker, said biomarker being IL-12.

In another particular embodiment, the first step of the first predictive method of the invention further comprises the determination in said biofluid sample from said subject of the levels of at least one additional biomarker, said biomarker being RANTES.

In another particular embodiment, the first step of the first predictive method of the invention further comprises the determination in said biofluid sample from said subject of the levels of at least one additional biomarker, said biomarker being EGF.

In a preferred embodiment, the first step of the first predictive method of the invention further comprises the determination in said biofluid sample from said subject of the levels of at least two additional biomarkers, said two additional biomarkers being IL-12 and RANTES.

In a preferred embodiment, the first step of the first predictive method of the invention further comprises the determination in said biofluid sample from said subject of the levels of at least two additional biomarkers, said two additional biomarkers being IL-12 and EGF.

In a preferred embodiment, the first step of the first predictive method of the invention further comprises the determination in said biofluid sample from said subject of the levels of at least two additional biomarkers, said two additional biomarkers being RANTES and EGF.

In a more preferred embodiment, the first step of the first predictive method of the invention further comprises the determination in said biofluid sample from said subject of the levels of IL-12, RANTES and EGF.

The terms "IL-12", "RANTES" and "EGF" have been described in detail in the context of the first diagnostic method of the invention and their definitions and particularities apply equally to the second diagnostic method of the invention.

The term "biofluid sample", as used in the context of the first predictive method of the invention, refers to any biofluid sample with the exemption of CSF. Preferably, the biofluid sample is blood, plasma, blood serum or saliva.

The level of biomarkers can be determined by any method known in the art suitable for the determination and quantification of a protein in a sample.

In a particular embodiment, the determination of the level of a biomarker is carried out by means of an immunoassay. In a preferred embodiment, the immunoassay is a chemiluminiscent enzyme immunoassay, more preferably a solid-phase chemiluminiscent enzyme immunoassay. These techniques have been described in detail in the context of the diagnostic methods of the invention.

The second step of the first predictive method of the invention comprises comparing the level of IP-10 with its reference value.

In particular embodiments, as aforementioned, the first predictive method of the invention further comprises comparing the level of said at least one additional biomarker selected from IL-12, RANTES and EGF with its reference value.

The term "reference value", has been previously defined in connection with the diagnostic methods of the invention. The reference value according to the predictive methods of the invention can be obtained from one or more subjects who are healthy at the moment of determining the likelihood that a subject develops AD, and who are known to have not developed AD. A subject is considered as not suffering from AD according to NINCDS-ADRDA criteria (McKhann G. et al., 1984, cited *supra*); particularly using the tests outlined in Table 2, or any other current or future medical criteria, such as the NIA-AA's criteria.

According to the first predictive method of the invention, decreased levels of IP-10 compared to the reference value is indicative of a high likelihood of the subject developing AD.

In particular embodiments, as aforementioned, in addition to decreased levels of IP-10 compared to the reference value, decreased levels of IL-12 and/or RANTES and/or EGF compared to their respective reference value is indicative of a high likelihood of the subject developing AD.

"Decreased" and "increased" levels of a biomarker have been described in detail in the context of the diagnostic methods of the invention.

In another particular embodiment, the first predictive method of the invention further comprises determining at least one demographic factor selected from age, sex and education level.

According to the first predictive method of the invention, in addition to decreased levels of IP-10 compared to the reference value, increased age and/or female gender and/or a low level of education is also indicative of high likelihood of the subject developing AD.

In a preferred embodiment, increased age (as years) presents an odds ratio (OD) of 1.10 for the likelihood of the subject developing AD.

In another preferred embodiment, female presents an OD of 2.25 in respect to male for the likelihood of the subject developing AD.

In another preferred embodiment, a level of education lower than basic education is also indicative of a high likelihood of the subject developing AD.

In another particular embodiment, the first predictive method of the invention further comprises determining the presence or absence of a nucleic acid sequence encoding ApoE4 in a sample containing nucleic acid from said subject. Accordingly, the presence of a nucleic acid sequence encoding ApoE4 is indicative of a high likelihood of the subject developing AD.

The term "ApoE4" has been described in detail in the context of the diagnostic methods of the invention and its particularities are equally applicable to the first predictive method of the invention.

### 2.1. Second predictive method

In another aspect, the invention relates to an *in vitro* method for determining the likelihood that a subject develops AD, hereinafter "the second predictive method of the invention", comprising:
i) determining in a biofluid sample from said subject the level of IL-5, and
ii) comparing the level of IL-5 with a reference value,
wherein decreased levels of IL-5 compared to the reference value is indicative of a high likelihood of the subject developing AD.

The terms "Alzheimer's disease", "subject", "biofluid sample", "level", and "IL-5" have been described in detail in the context of the diagnostic methods of the invention and their definitions and particularities apply equally to the second predictive method of the invention. The term "reference value" has been described in detail in the context of the first predictive method of the invention and its definitions and particularities apply equally to the second predictive method of the invention.

In a particular embodiment of the second predictive method of the invention, the subject is a subject suffering from prodromal AD or MCI.

The first step of the second predictive method of the invention comprises the determination in a biofluid sample from said subject of the level of IL-5.

In a particular embodiment, the first step of the second predictive method of the invention further comprises the determination in said biofluid sample from said subject of the levels of at least one additional biomarker, said biomarker being IL-12.

In another particular embodiment, the first step of the second predictive method of the invention further comprises the determination in said biofluid sample from said subject of the levels of at least one additional biomarker, said biomarker being RANTES.

In another particular embodiment, the first step of the second predictive method of the invention further comprises the determination in said biofluid sample from said subject of the levels of at least one additional biomarker, said biomarker being EGF.

In a preferred embodiment, the first step of the second predictive method of the invention further comprises the determination in said biofluid sample from said subject of the levels of at least two additional biomarkers, said two additional biomarkers being IL-12 and RANTES.

In a preferred embodiment, the first step of the second predictive method of the invention further comprises the determination in said biofluid sample from said subject of the levels of at least two additional biomarkers, said two additional biomarkers being IL-12 and EGF.

In a preferred embodiment, the first step of the second predictive method of the invention further comprises the determination in said biofluid sample from said subject of the levels of at least two additional biomarkers, said two additional biomarkers being RANTES and EGF.

In a more preferred embodiment, the first step of the second predictive method of the invention further comprises the determination in said biofluid sample from said subject of the levels of IL-12, RANTES and EGF.

The terms "IL-12", "RANTES" and "EGF" have been described in detail in the context of the first diagnostic method of the invention and their definitions and particularities apply equally to the second diagnostic method of the invention.

The level of biomarkers can be determined by any method known in the art suitable for the determination and quantification of a protein in a sample.

In a particular embodiment, the determination of the level of a biomarker is carried out by means of an immunoassay. In a preferred embodiment, the immunoassay is a chemiluminiscent enzyme immunoassay, more preferably a solid-phase chemiluminiscent enzyme immunoassay. These techniques have been described in detail in the context of the diagnostic methods of the invention.

The second step of the second predictive method of the invention comprises comparing the level of IL-5 with its reference value.

In particular embodiments, as aforementioned, the second predictive method of the invention further comprises comparing the level of said at least one additional biomarker selected from IL-12, RANTES and EGF with its reference value.

According to the second predictive method of the invention, decreased levels of IL-5 compared to the reference value is indicative of a high likelihood of the subject developing AD.

In particular embodiments, as aforementioned, in addition to decreased levels of IL-5 compared to the reference value, decreased levels of IL-12 and/or RANTES and/or EGF compared to their respective reference value is indicative of a high likelihood of the subject developing AD.

"Decreased" and "increased" levels of a biomarker have been described in detail in the context of the diagnostic methods of the invention.

In another particular embodiment, the second predictive method of the invention further comprises determining at least one demographic factor selected from age, sex and education level.

According to the second predictive method of the invention, in addition to decreased levels of IL-5 compared to the reference value, increased age and/or female gender and/or a low level of education is also indicative of a high likelihood of the subject developing AD.

In a preferred embodiment, increased age (as years) presents an odds ratio (OD) of 1.08 for the likelihood of the subject developing AD.

In another preferred embodiment, female presents an OD of 2.06 in respect to male for the likelihood of the subject developing AD.

In another preferred embodiment, a level of education lower than basic education is also indicative of a high likelihood of the subject developing AD.

In another particular embodiment, the second predictive method of the invention further comprises determining the presence or absence of a nucleic acid sequence encoding ApoE4 in a sample containing nucleic acid from said subject. Accordingly, the presence of a nucleic acid sequence encoding ApoE4 is indicative of a high likelihood of the subject developing AD.

The term "ApoE4" has been described in detail in the context of the diagnostic methods of the invention and its particularities are equally applicable to the second predictive method of the invention.

### 2.1. Third predictive method

In another aspect, the invention relates to an *in vitro* method for determining the likelihood that a subject develops AD, hereinafter "the third predictive method of the invention", comprising:
i) determining in a first biofluid sample from said subject the level of IP-10,
ii) determining in a second biofluid sample from said subject the level of IL-5, and
iii) comparing the level of IP-10 and IL-5 with their corresponding reference values,
wherein said first biofluid is not CSF,
wherein said first biofluid sample and said second biofluid sample are the same or different, and
wherein decreased levels of IP-10 compared to its reference value and decreased levels of IL-5 compared to its reference value is indicative of a high likelihood of the subject developing AD

The terms "Alzheimer's disease", "subject", "level", "IP-10" and "IL-5" have been described in detail in the context of the diagnostic methods of the invention and their definitions and particularities apply equally to the third predictive method of the invention. The term "reference value" has been described in detail in the context of the first predictive method of the invention and its definitions and particularities apply equally to the third predictive method of the invention.

In a particular embodiment of the second predictive method of the invention, the subject is a subject suffering from prodromal AD or MCI.

The first step (i) of the third predictive method of the invention comprises the determination in a biofluid sample from said subject of the level of IP-10.

The second step (ii) of the third predictive method of the invention comprises the determination in a biofluid sample from said subject of the level of IL-5.

The first and second steps of the third predictive method of the invention can be performed in any order. Accordingly, the first step (i) can be carried out before the second step (ii), or the second step (ii) can be carried out before the first step (i).

The term "first biofluid sample", as used in the context of the third predictive method of the invention, refers to any biofluid sample with the exception of CSF. Preferably, the first biofluid sample is blood, plasma, blood serum or saliva.

It will be recognisable for the person skilled in the art that said first biofluid sample may be the same as said second biofluid sample or, alternatively, that said first biofluid sample may be different from said second biofluid sample.

In a particular embodiment, the first step of the third predictive method of the invention further comprises the determination in said biofluid sample from said subject of the levels of at least one additional biomarker, said biomarker being IL-12.

In another particular embodiment, the first step of the third predictive method of the invention further comprises the determination in said biofluid sample from said subject of the levels of at least one additional biomarker, said biomarker being RANTES.

In another particular embodiment, the first step of the third predictive method of the invention further comprises the determination in said biofluid sample from said subject of the levels of at least one additional biomarker, said biomarker being EGF.

In a preferred embodiment, the first step of the third predictive method of the invention further comprises the determination in said biofluid sample from said subject of the levels of at least two additional biomarkers, said two additional biomarkers being IL-12 and RANTES.

In a preferred embodiment, the first step of the third predictive method of the invention further comprises the determination in said biofluid sample from said subject of the levels of at least two additional biomarkers, said two additional biomarkers being IL-12 and EGF.

In a preferred embodiment, the first step of the third predictive method of the invention further comprises the determination in said biofluid sample from said subject of the levels of at least two additional biomarkers, said two additional biomarkers being RANTES and EGF.

In a more preferred embodiment, the first step of the third predictive method of the invention further comprises the determination in said biofluid sample from said subject of the levels of IL-12, RANTES and EGF.

The terms "IL-12", "RANTES" and "EGF" have been described in detail in the context of the diagnostic methods of the invention and their definitions and particularities apply equally to the second predictive method of the invention.

The level of biomarkers can be determined by any method known in the art suitable for the determination and quantification of a protein in a sample.

In a particular embodiment, the determination of the level of a biomarker is carried out by means of an immunoassay. In a preferred embodiment, the immunoassay is a chemiluminiscent enzyme immunoassay, more preferably a solid-phase chemiluminiscent enzyme immunoassay. These techniques have been described in detail in the context of the first diagnostic method of the invention.

The third step (iii) of the third predictive method of the invention comprises comparing the level of IP-10 and IL-5 with their corresponding reference values.

In particular embodiments, as aforementioned, the third predictive method of the invention further comprises comparing the level of said at least one additional biomarker selected from IL-12, RANTES and EGF with its reference value.

According to the third predictive method of the invention, decreased levels of IP-10 compared to its reference value and decreased levels of IL-5 compared to its reference value is indicative of a high likelihood of the subject developing AD.

In particular embodiments, as aforementioned, in addition to decreased levels of IP-10 compared to the reference value and decreased levels of IL-5 compared to the reference value, decreased levels of IL-12 and/or RANTES and/or EGF compared to their respective reference value is indicative of a high likelihood of the subject developing AD.

"Decreased" and "increased" levels of a biomarker have been described in detail in the context of the first diagnostic method of the invention.

In another particular embodiment, the third diagnostic method of the invention further comprises determining at least one demographic factor selected from age, sex and education level.

According to the third diagnostic method of the invention, in addition to decreased levels of IP-10 compared to the reference value and decreased levels of IL-5 compared to the reference value, increased age and/or female gender and/or a low level of education is also indicative of a high likelihood of the subject developing AD.

In a preferred embodiment, increased age (as years) presents an odds ratio (OD) of 1.08 for the likelihood of the subject developing AD.

In another preferred embodiment, female presents an OD of 2.04 in respect to male for the likelihood of the subject developing AD.

In another preferred embodiment, a level of education lower than basic education is also indicative of a high likelihood of the subject developing AD.

In another particular embodiment, the third diagnostic method of the invention further comprises determining the presence or absence of a nucleic acid sequence encoding ApoE4 in a sample containing nucleic acid from said subject. Accordingly, the presence of a nucleic acid sequence encoding ApoE4 is indicative of a high likelihood of the subject developing AD.

The term "ApoE4" has been described in detail in the context of the first diagnostic method of the invention and its particularities are equally applicable to the second predictive method of the invention.

### 3. Methods for the treatment or prevention of AD

According to the previous aspects of the invention, the determination of a series of markers allows the identification of subjects who are suffering from AD and who have a high probability of developing AD. Therefore, this information can be used for the identification of subjects which would benefit from the treatment with a therapy aimed at ameliorating, stabilising and/or preventing the appearance AD.

Thus, in another aspect, the invention relates to a method for the treatment of AD in a subject in need thereof, hereinafter "the therapeutic method of the invention", which comprises the administration to the subject a drug useful for the treatment of AD, wherein the subject has been selected by a diagnostic method according to the invention.

In another aspect, the invention relates to a method for the prevention of AD in a subject in need thereof, hereinafter "the method of prevention of the invention", which comprises the administration to the subject of a drug useful for the prevention of AD, wherein the subject has been selected by a predictive method according to the invention.

The term "Alzheimer's disease" has been described in detail in the context of the first diagnostic method of the invention and its definitions and particularities apply equally to the therapeutic method of the invention.

The expression "treatment and/or prevention", as used herein, refers to both therapeutic measures and prophylactic or preventive measures, wherein the object is to prevent or slow down (lessen) an undesired physiological change or disorder, such as AD. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment and/or prevention" can also mean prolonging survival as compared to expected survival if not receiving treatment. Those in need of treatment include those already with the condition or disorder as well as those prone to have the condition or disorder or those in which the condition or disorder is to be prevented.

The term "therapy for the treatment of AD", as used herein, refers to any therapy directed to alleviate the symptoms, diminish the extent of the disease, stabilize (i.e., not worsen) the state of disease, delay or slow disease progression, ameliorate or palliate the disease state, or achieve remission (whether partial or total) of the disease.

There are two main types of therapies used to treat AD: cholinesterase inhibitors and NMDA receptor antagonists. Cholinesterase inhibitors include donepezil hydrochloride (Aricept), rivastigmine (Exelon) and galantamine (Reminyl) and are directed to prevent the breakdown of acetylcholine by acetylcholinesterase, improving the function of brain cells. The NMDA receptor antagonist memantine (Ebixa) bind to NMDA receptors on brain cells blocking the activity of glutamate, which is released in increased amounts in brain cells of patients with AD.

Other therapies described for the treatment and/or prevention of AD are directed to reduce the toxic effects caused by the deposition of the amyloid beta peptide β-amyloid, AB or Aβ) that comes from amyloid precursor protein (APP). For example, WO09087568A describes compositions including compounds such as kercetin, bipigenine and kaemferol that reduce neuronal death caused by exposure to Aβ peptide. US5948800A describes the use of drugs to prevent or treat AD, these drugs contain the active compound 2-phenyl-1, 2-benzisoselenazol-3 (2H)-one, whose effect is based on the reduction of neuronal toxicity caused by the peptide Aβ. US2002102259 describes a method for inhibiting Aβ peptide effects in the brain of an animal comprising administering a compound that effectively modulates the activity of CD45.

Additionally, other therapeutic agents with anti-inflammatory activity are also contemplated within the scope of the methods for the treatment or prevention of AD of the invention. These include, without limitation, prednisone, conjugated oestrogens, transdermal selegiline, rofecoxib, celecoxib, acetyl-L-carnitine, ginkgo biloba extract, idebenone, nicergoline, propentofylline, and non-steroidal anti-inflammatory drugs (NSAIDs), such as aspirin, ibuprofen and naproxen.

In a particular embodiment, the therapy for the treatment of AD that is administered according to the third method of the invention is a cholinesterase inhibitor. In another particular embodiment, the therapy for the treatment of AD that is administered according to the third method of the invention is a NMDA receptor antagonist.

### EXAMPLES

### Materials and methods

### Samples

217 serum samples were collected from individuals diagnosed as healthy controls (HC) and 94 serum samples from probable Alzheimer's disease (AD). HC individuals were recruited from neurology departments of hospitals and primary attention clinics and showed not referred memory decline and cognitive impairment. AD individuals were diagnosed by neurologist recruited from neurology departments of three big hospitals (University Hospital *La Paz,* Madrid; University Hospital *Virgen de Las Nieves* and Clinic Hospital, Granada, Spain). AD individuals were diagnosed by Petersen scale of cognitive impairment including several neuropsycological tests such as MMSE, and other worldwide used scales such as Hachinski, Photo-test and others. The result of different test was a CDS scale where 0 for HC, and 1 or more for AD. The blood samples were recollected and processed to obtain serum by centrifugation in pre-treated tubes, and kept frozen at -80°C until biomarker detection and quantitation.

### Biomarker detection

Serum samples were analysed for 41 cytokines and chemokines using a commercially available Cytokine Human 41-plex panel (Millipore). Samples were measured in duplicates and according to the manufacturer's recommendations, using the antibody bead mix with biotinylated detection antibody followed by streptavidin-phycoerythrin. The plate was read using the Luminex platform (Milliplex), and data were collected for 100 beads per cytokine from each well. Cytokine concentrations were calculated using Milliplex Analyst software with a five parameter curve-fitting algorithm applied for standard curve calculations.

### Statistics

### STATA software was used for all statistics analysis

### - Univariate analysis

The comparison of the distribution or median of the biomarkers level was compared in both groups (healthy control or probable AD) by Mann-Whitney U test. Statistically significant results were considered when p-value was lower than 0.05. Results of biomarker serum levels were adjusted for age, sex and education level, and discrimination capacity between both groups was calculated by the area under the ROC curve of each of the markers for diagnosis of AD. The diagnostic rate of biomarkers was calibrated by Hosmer-Lemeshow test.

### - Multivariate analysis

To assess the association of each of the markers with diagnosis of AD, there were two statistical approaches. First, a regression model was generated for each of the markers after adjusting for age, sex and education level. Additionally, the area under the ROC curve of each of the markers was calculated for diagnosis of AD.

To examine the predictive value of different markers, a strategy consisting of analysis using multivariate logistic regression models was developed, having the state of the patient (healthy control or probable AD) as dependent variable and subsets of markers selected according to the maximum Youden index criteria (sum of sensitivity and specificity) as independent variables.

The modelling strategy began with the definition of a Maximum Likelihood Estimation model with the markers and adjusted for the age, sex and level of education. This was followed by non-automatic backward modelling, where markers with a lower predictive ability not statistically significant (lower likelihood ratio) were excluded sequentially from the model. With this strategy, a final model with markers who were significantly associated to the diagnosis of probable AD was reached.

The final model was analysed according to its capacity to discriminate between patients with probable AD and healthy controls (area under the ROC curve). It is considered a model has good capacity of discrimination when the area under the ROC curve is above 0.7. The calibration of the final model was also evaluated by comparing the odds of AD estimated by the model and those observed in the sample (Hosmer-Lemeshow test).

### Example 1: Biomarkers with diagnostic capacity

The inventors analysed serum levels of 41 cytokines to find one or various markers with capacity to discriminate between heathy controls and Alzheimer's disease patients. First, all biomarkers were individually analysed to determine which ones could have diagnostic capacity. Subsequently, the inventors studied combinations of biomarkers to find out if their diagnostic capacity could be increased.

The criterion for determining the cut-off point for each marker was to maximise the Youden index (sum of sensitivity and specificity). In medical diagnostics, the sensitivity of a test is the ability of the test to identify correctly those subjects suffering from a disease (true positive rate), whereas the specificity of a test is the ability of the test to identify correctly those subjects not suffering with the disease (true negative rate). Once the optimal cut-off point was set, the minimum diagnostic performance threshold to select the marker for the subsequent modelling phase was established. The Youden index was set at 110 and one of sensitivity or specificity was set to be higher than 70. With these parameters, values lower than the optimised cut-off points for two markers, IL-5 and IP-10, were associated with an increased risk of AD.

In respect to IL-5, comparison of the distribution (median) of IL-5 between AD and healthy control groups was significant (Table 1, Figs. 1 and 2). The selected cut-off was based on the Youden index for this biomarker, such that a score lower than this cut-off point is considered as a positive result for the biomarker. This point provides 51.1% sensitivity on the 94 AD cases and 83.0% specificity on the 217 HC cases.

**Table 1. Distribution of IL-5 plasma levels (pg/ml) between AD and HC groups.**

| | **AD** | | **HC** | | **Total** | | **p**-**value** |
|---|---|---|---|---|---|---|---|
| | P₅₀ (P₂₅-P₇₅) | Min-Max | P₅₀ (P₂₅-P₇₅) | Min-Max | P₅₀ (P₂₅-P₇₅) | Min-Max | |
| **IL-5** | 1,06 (0,80-1,50) | 0,4-12,8 | 1,42 (1,17-2,00) | 0,5-15,7 | 1,40 (1,06-1,80) | 0,4-15,7 | <0,001 |

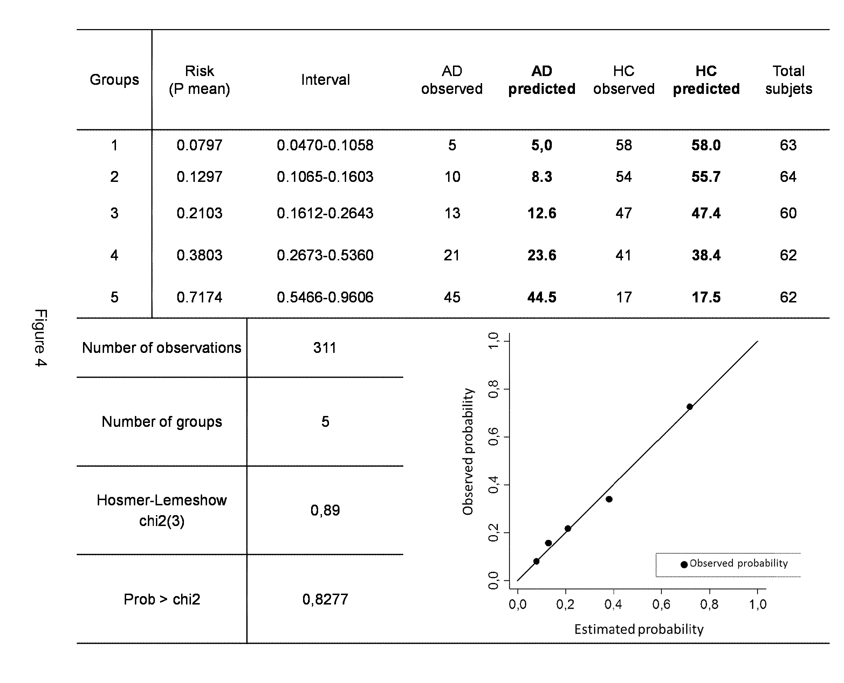

Table 2 shows the results of the capacity of the model to discriminate and its calibration in conjunction with demographic characteristics such as age, sex and level of education. IL-5 had an area under the ROC curve of 0.804 (Figure 3). To evaluate the calibration of the model, i.e. the similarity between the probabilities predicted by the model and the observed probabilities, the Hosmer-Lemeshow test was used. Figure 4 shows the table for quintiles of the predicted probability. For example, in the quintile 1 (Group 1), the probability predicted by the model is between 0.0470 and 0.1058, with a mean of 0.0797. This group had 63 individuals of whom 5 were Alzheimer's and the model predicts 5.0 individuals, demonstrating a good calibration in this quintile. The same applies to the rest of quintiles. Global analysis using the Chi-square test shows that the differences between the observed frequencies and those predicted by the model are not statistically significant. The relationship between the observed probability and the probability estimated by the model is shown graphically in figure 4 by dividing the sample into quintiles based on the predicted probability.

**Table 2. Discrimination capacity of IL-5 in conjunction with demographic factors (age, gender and education level).**

| **Univariate IL-5** | | | | | |
|---|---|---|---|---|---|
| | | | *ICI (95%)* | | |
| | | *OR* | *Lower* | *Upper* | *P-Value* |
| | **Age (Years)** | 1,08 | 1,04 | 1,14 | 0,001 |
| **Gender Female (Ref. Male)** | | 2,06 | 1,13 | 3,76 | 0,019 |
| **Education (Ref. Low Level)** | **Basic** | 0,14 | 0,05 | 0,38 | <0,001 |
| | **Primary/Secondary** | 0,12 | 0,04 | 0,35 | <0,001 |
| | **High level** | 0,11 | 0,03 | 0,36 | <0,001 |
| | **IL-5** | 5,15 | 2,81 | 9,42 | <0,001 |

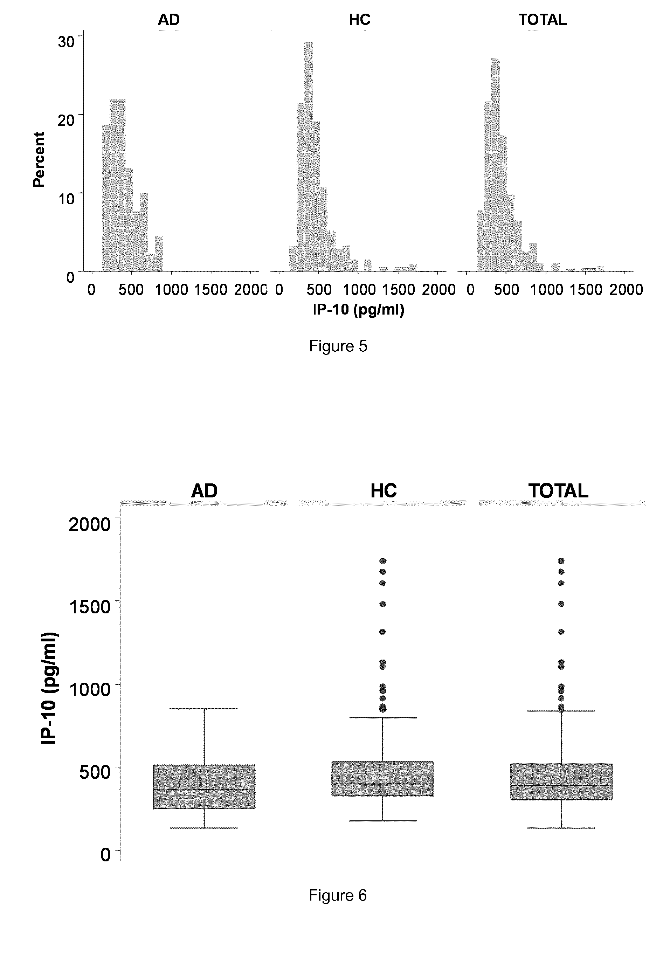

In respect to IP-10, comparison of the distribution of IP-10 (median) among AD and HC groups was significant (Table 3, Figs. 5 and 6). The selected cut-off was based on the Youden index for this biomarker, such that a score lower than this cut-off point is considered as a positive result for the biomarker. This point provides 35.1% sensitivity on the 94 AD cases and 84.3% specificity on the 217 HC cases.

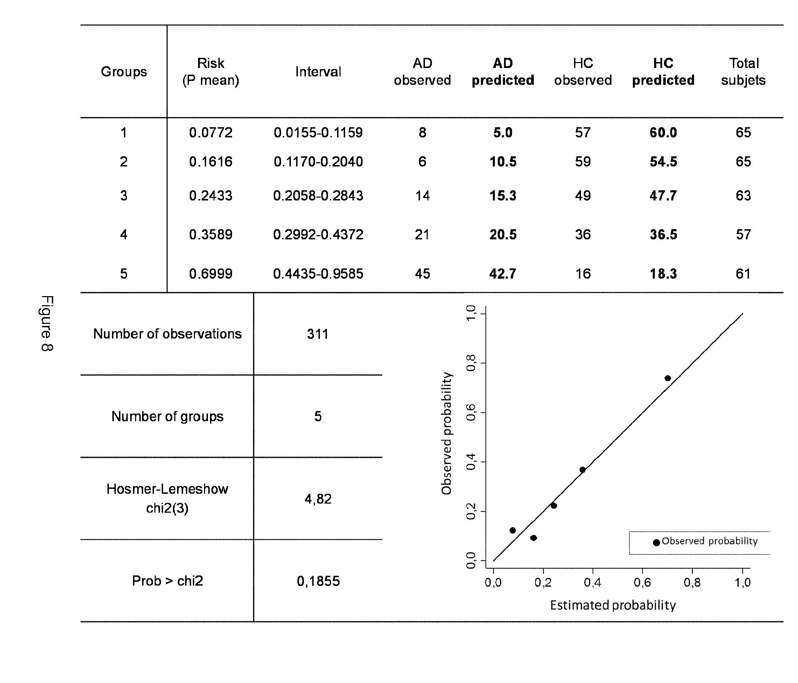

Table 4 shows the results of the capacity of the model to discriminate and its calibration in conjunction with demographic characteristics such as age, sex and level of education. IP-10 had an area under the ROC curve of 0.795 (Fig. 7) and an excellent calibration showing a p-value of 0.19 for the Hosmer-Lemeshow test (Fig. 8).

**Table 3. Distribution of IP-10 plasma levels (pg/ml) between AD and HC groups.**

| | **AD** | | **HC** | | **Total** | | **p**-**value** |
|---|---|---|---|---|---|---|---|
| | P₅₀ (P₂₅-P₇₅) | Min-Max | P₅₀ (P₂₅-P₇₅) | Min-Max | P₅₀ (P₂₅-P₇₅) | Min-Max | |
| **IP-10** | 366,5 (252-519) | 137-9520 | 401 (331-532) | 178-2839 | 395 (307-532) | 137-9520 | 0,032 |

**Table 4. Discrimination capacity of IP-10 in conjunction with demographic factors (age, gender and education level).**

| **Univariate IP-10** | | | | | |
|---|---|---|---|---|---|
| | | | *ICI (95%)* | | *P-Value* |
| | | *OR* | *Lower* | *Upper* | |
| | **Age (Years)** | 1,10 | 1,05 | 1,16 | <0,001 |
| | **Gender Female (Ref. Male)** | 2,25 | 1,24 | 4,07 | 0,008 |
| **Education (Ref. Low Level)** | **Basic** | 0,17 | 0,06 | 0,47 | 0,001 |
| | **Primary/Secondary** | 0,14 | 0,05 | 0,39 | <0,001 |
| | **High level** | 0,09 | 0,03 | 0,31 | <0,001 |
| | **IP-10** | 4,51 | 2,30 | 8,82 | <0,001 |

**Table 5. Discrimination capacity of themultivariate model adjusted by demographic factors (age, gender and education level).**

| **Multivariate statistical model** | | | | | |
|---|---|---|---|---|---|
| | | | *ICI (95%)* | | *P-Value* |
| | | *OR* | *Lower* | *Upper* | |
| | **Age (Years)** | 1,10 | 1,04 | 1,16 | 0,001 |
| | **Gender Female (Ref. Male)** | 2,04 | 1,02 | 4,06 | 0,043 |
| **Education (Ref. Low Level)** | **Basic** | 0,13 | 0,04 | 0,46 | 0,001 |
| | **Primary/Secondary** | 0,08 | 0,02 | 0,29 | <0,001 |
| | **High level** | 0,05 | 0,01 | 0,24 | <0,001 |
| **Marker** | **EGF** | 4,91 | 1,63 | 14,82 | 0,005 |
| | **IL-12** | 4,11 | 1,71 | 9,88 | 0,002 |
| | **IL-5** | 2,92 | 1,36 | 6,27 | 0,006 |
| | **IP-10** | 5,02 | 2,26 | 11,14 | <0,001 |
| | **RANTES** | 3,62 | 1,54 | 8,51 | 0,003 |

### Example 2: Multivariate model

The inventors found three additional markers (EGF, IL-12 and RANTES) that, taken together with IL-5 and IP-10, increased their diagnostic capacity (Table 5). The discrimination of the model, i.e. rather than discriminating between AD patients and HC, has been evaluated through the area under the curve (AUC) ROC of the probability predicted by the model (Fig. 9), resulting in that the model had a very good capacity for discrimination (AUC = 0.88).

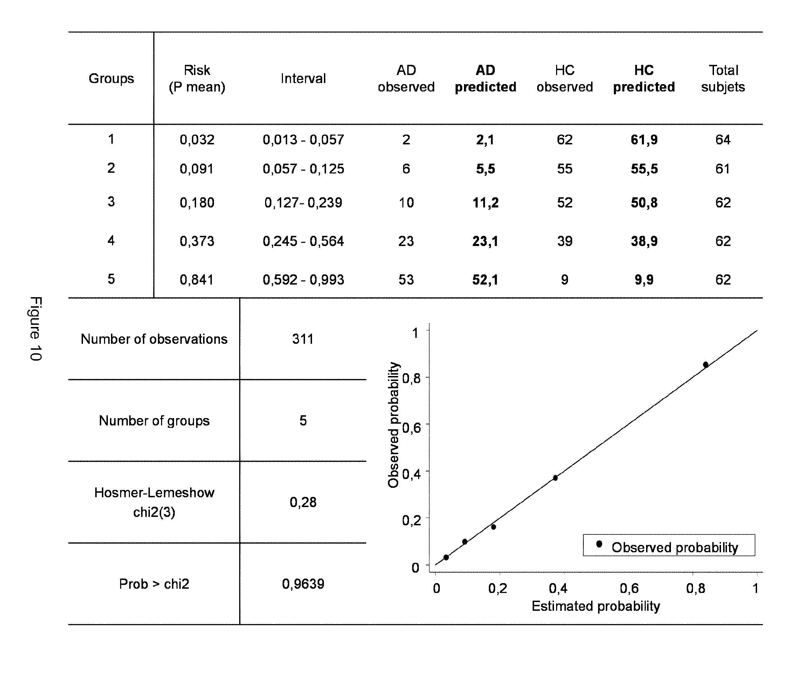

Figure 10 shows the table and graphics from the Hosmer-Lemeshow test. There is an excellent calibration (p-value = 0.96), so according to risk levels estimated by the model, the observed frequency of individuals with Alzheimer's disease coincides with the frequency predicted by the multivariate model.

## Claims

1. An *in vitro* method for diagnosing Alzheimer's disease (AD) in a subject comprising:
i) determining in a biofluid sample from said subject the level of IP-10, and
ii) comparing the level of IP-10 with a reference value,
wherein said biofluid is not cerebrospinal fluid (CSF), and
wherein decreased levels of IP-10 compared to the reference value is indicative of said subject suffering from AD.

2. An *in vitro* method for diagnosing AD in a subject comprising:
i) determining in a biofluid sample from said subject the level of IL-5, and
ii) comparing the level of IL-5 with a reference value,
wherein decreased levels of IL-5 compared to the reference value is indicative of said subject suffering from AD.

3. An *in vitro* method for diagnosing AD in a subject comprising:
i) determining in a first biofluid sample from said subject the level of IP-10,
ii) determining in a second biofluid sample from said subject the level of IL-5, and
iii) comparing the level of IP-10 and IL-5 with their corresponding reference values,
wherein said first biofluid is not CSF,
wherein said first biofluid sample and said second biofluid sample are the same or different, and
wherein decreased levels of IP-10 compared to its reference value and decreased levels of IL-5 compared to its reference value is indicative of said subject suffering from AD.

4. An *in vitro* method for determining the likelihood that a subject develops AD, comprising:
i) determining in a biofluid sample from said subject the level of IP-10, and
ii) comparing the level of IP-10 with a reference value,
wherein said biofluid is not CSF, and
wherein decreased levels of IP-10 compared to the reference value is indicative of a high likelihood of the subject developing AD.

5. An *in vitro* method for determining the likelihood that a subject develops AD, comprising:
i) determining in a biofluid sample from said subject the level of IL-5, and
ii) comparing the level of IL-5 with a reference value,
wherein decreased levels of IL-5 compared to the reference value is indicative of a high likelihood of the subject developing AD.

6. An *in vitro* method for determining the likelihood that a subject develops AD, comprising:
i) determining in a first biofluid sample from said subject the level of IP-10,
ii) determining in a second biofluid sample from said subject the level of IL-5, and
iii) comparing the level of IP-10 and IL-5 with their corresponding reference values,
wherein said first biofluid is not CSF,
wherein said first biofluid sample and said second biofluid sample are the same or different, and
wherein decreased levels of IP-10 compared to its reference value and decreased levels of IL-5 compared to its reference value is indicative of a high likelihood of the subject developing AD.

7. The *in vitro* method for diagnosing AD in a subject according to any of claims 1 to 3, or the *in vitro* method for determining the likelihood that a subject develops AD according to any of claims 4 to 6, further comprising
a) determining in said biofluid sample from said subject the levels of at least one additional biomarker selected from the group consisting of IL-12, RANTES and EGF, and
b) comparing the level of said additional biomarker with its reference value,
wherein decreased levels of IL-12 compared to its reference value and/or decreased levels of RANTES compared with its reference value and/or decreased levels of EGF compared with its reference value are indicative of the subject suffering from AD, or wherein decreased levels of IL-12 compared to its reference value and/or decreased levels of RANTES compared with its reference value and/or decreased levels of EGF compared with its reference value are indicative of a high likelihood of the subject developing AD.

8. The *in vitro* method for diagnosing AD in a subject or the *in vitro* for determining the likelihood that a subject develops AD according to claim 7, wherein step (a) comprises determining the levels of at least two additional biomarkers selected from the group consisting of IL-12, RANTES and EGF.

9. The *in vitro* method for diagnosing AD in a subject or the *in vitro* method for determining the likelihood that a subject develops AD according to claim 8, wherein step (a) comprises determining the levels of IL-12, RANTES and EGF.

10. The *in vitro* method for diagnosing AD in a subject according to any of claims 1 to 3 or 7 to 9, or the *in vitro* method for determining the likelihood that a subject develops AD according to any of claims 4 to 9, wherein said subject is a subject suffering from prodromal AD or mild cognitive impairment.

11. The *in vitro* method for diagnosing AD in a subject according to any of claims 1 to 3 or 7 to 10, or the *in vitro* method for determining the likelihood that a subject develops AD according to any of claims 4 to 10, further comprising determining at least one demographic factor selected from age, gender and education level.

12. The *in vitro* method for diagnosing AD in a subject according to any of claims 1 to 3 or 7 to 11, or the *in vitro* method for determining the likelihood that a subject develops AD according to any of claims 4 to 11, further comprising determining the presence or absence of a nucleic acid sequence encoding apolipoprotein E type 4 isoform (ApoE4) in a sample containing nucleic acid from said subject, wherein the presence of a nucleic acid sequence encoding said ApoE4 is indicative of the subject suffering from AD, or wherein the presence of a nucleic acid sequence encoding said ApoE4 is indicative of a high likelihood of the subject developing AD.

13. The *in vitro* method for diagnosing AD in a subject according to any of claims 1 to 3 or 7 to 12, or the *in vitro* method for determining the likelihood that a subject develops AD according to any of claims 4 to 12, wherein the determination of the level of IP-10, IL-5, IL-12, RANTES and/or EGF is carried out by means of an immunoassay.

14. A method for the treatment of AD in a subject in need thereof, which comprises the administration to the subject a drug useful for the treatment of AD, wherein the subject has been selected by a method according to any of claims 1 to 3 or 7 to 13.

15. A method for the prevention of AD in a subject in need thereof, which comprises the administration to the subject of a drug useful for the prevention of AD, wherein the subject has been selected by a method according to any of claims 4 to 13.
